Europäisches Patentamt

European Patent Office

Office européen des brevets

(11) Veröffentlichungsnummer: **0 000 495**
**B1**

## (12) EUROPÄISCHE PATENTSCHRIFT

(21) Anmeldenummer: **78100358.7**

(22) Anmeldetag: **11.07.78**

(51) Int. Cl.³: **C 07 C 143/66**

(54) **Verfahren zur Herstellung von 1-Amino-8-naphthol-3,6-disulfonsäure (H - Säure)**

(30) Priorität: **16.07.77 DE 2732266**

(43) Veröffentlichungstag der Anmeldung:
**07.02.79 Patentblatt 79/03**

(45) Bekanntmachung des Hinweises auf die Patenterteilung:
**26.11.80 Patentblatt 80/24**

(84) Benannte Vertragsstaaten:
**BE CH DE FR GB**

(56) Entgegenhaltungen:
**US A - 1670406**

(73) Patentinhaber: **BAYER AG**
**Zentralbereich Patente, Marken und Lizenzen**
**D - 5090 Leverkusen 1, Bayerwerk (DE)**

(72) Erfinder: **Schössler, Willi, Dr.**
**Hahnenweg 2**
**D - 5000 Köln 80 (DE)**
**Pütter, Rolf, Dr.**
**Poststrasse 7**
**D - 4000 Düsseldorf 1 (DE)**
**Behre, Horst, Dr.**
**Im Hellsiefen 4**
**D - 5068 Odenthal (DE)**

Courier Press, Leamington Spa, England.

# 0 000 495

Verfahren zur Herstellung von 1-Amino-8-naphthol-3,6-disulfonsäure (H-Säure)

Die vorliegende Erfindung betrifft ein Verfahren zur Herstellung von 1-Amino-8-naphthol-3,6-disulfonsäure (H-Säure) als Monoalkalisalz aus 1-Naphthylamino-3,6,8-trisulfonsäure durch alkalische Druckhydrolyse.

1-Amino-8-naphthol-3,6-disulfonsäure, die häufig auch als H-Säure bezeichnet wird, ist ein wichtiges Zwischenprodukt zur Herstellung von Farbstoffen (s. Ullmanns Enzyklopädie der Technischen Chemie, 3. Auflage, 12. Band, S. 621).

Aus FIAT Final Report Nr. 1016, S. 32 bis 39 ist bekannt, daß man H-Säure wie folgt herstellen kann: Naphthalin wird mit Schwefelsäuremonohydrat (= 100%iger $H_2SO_4$) und 65%igem Oleum unter Einhaltung eines bestimmten Temperaturprogrammes und abgestufter Zugabe von Schwefelsäuremonohydrat und Oleum zu einem Naphthalin-trisulfonsäure-Isomerengemisch umgesetzt, das mit Mischsäure nitriert wird. Nach Verdünnung mit Wasser, Austreibung der nitrosen Gase und Abtrennung der Schwefelsäure als Calciumsulfat wird das Isomerengemisch der Nitronaphthalintrisulfonsäuren mit Eisen reduziert und dann gelöste Eisensalze mit Magnesiumoxid gefällt und abgetrennt. Durch Zugabe von Steinsalz und Salzsäure wird das saure Calcium-Natrium-Salz der T-Säure (1-Naphthylamin-3,6,8-trisulfonsäure) ausgefällt, das abfiltriert und mehrmals gewaschen wird. Dieses Salz wird in Waschwasser eingetragen und mit Soda versetzt. Dann wird von der ausgefallenen Kreide abgepreßt und die Salzlösung eingeengt. Die eingeengte Trinatriumsalzlösung der T-Säure wird mit 50%iger Natronlauge unter Druck umgesetzt. Anschließend wird zuerst Schwefelsäure, dann Wasser zugegeben und schließlich die H-Säure als Mononatriumsalz durch Filtration, Wäsche und Trocknung gewonnen.

Bei diesem Verfahren werden bei der alkalischen Druckhydrolyse der T-Säure erhebliche Mengen von Nebenprodukten gebildet, z.B. die 1-Amino-6-naphthol-3,8-disulfonsäure, ein unter dem Namen W-Säure bekanntes Isomeres der H-Säure, und die 1,8-Dihydroxy-naphthalin-3,6-disulfonsäure, ein unter dem Namen Chromotrop-Säure bekanntes Folgeprodukt der H-Säure. Die Ausbeute an H-Säure beträgt in dem zuvor geschilderten Verfahren im allgemeinen nur 70 bis 72%, bezogen auf eingesetzte T-Säure.

Es wurde nun ein Verfahren zur Herstellung von 1-Amino-8-naphthol-3,6-disulfonsäure-Monoalkalisalzen durch Umsetzung von 1-Naphthylamin-3,6,8-trisulfonsäure und/oder deren Salzen und/oder von Naphthylamin-trisulfonsäure-Isomerengemischen und/oder deren Salzen mit Alkalihydroxidlösung bei erhöhtem Druck und erhöhter Temperatur und Abscheidung von 1-Amino-8-naphthol-3,6-disulfonsäure-Monoalkalisalzen durch Ansäuern gefunden, das dadurch gekennzeichnet ist, daß man die Umsetzung in Gegenwart von Alkoholen und/oder Alkoholaten und in Gegenwart von Salpetersäure, Salpetriger Säure, Stickstoffoxiden, Nitraten, Nitriten, Nitroverbindungen oder Nitrosoverbindungen durchführt.

In das erfindungsgemäße Verfahren kann 1-Naphthylamin-3,6,8-trisulfonsäure (T-Säure) in reiner Form und/oder in Form von Naphthylamin-trisulfonsäure-Isomerengemischen eingesetzt werden. Die Naphthylamin-trisulfonsäure-Isomerengemische enthalten im allgemeinen über 65 Gew.-% 1-Naphthylamin-3,6,8-trisulfonsäure, bezogen auf die Gesamtmenge diazotierbarer Substanz. Vorzugsweise werden im Falle des Einsatzes von Naphthylamin-trisulfonsäure-Isomerengemischen solche eingesetzt, die 70 bis 90 Gew.-% 1-Naphthylamin-3,6,8-trisulfonsäure enthalten. Ein besonders bevorzugt einzusetzendes Naphthylamin-trisulfonsäure-Isomerengemisch enthät

| 75 bis | 85 Gew.-% | 1-Naphthylamin-3,6,8-trisulfonsäure |
|---|---|---|
| 5 bis | 15 Gew.-% | 1-Naphthylamin-3,5,7-trisulfonsäure |
| 1 bis | 10 Gew.-% | 1-Naphthylamin-4,6,8-trisulfonsäure |
| 0,5 bis | 5 Gew.-% | 1-Naphthylamin-2,5,7-trisulfonsäure |
| 0,1 bis | 2 Gew.-% | 2-Naphthylamin-3,5,7-trisulfonsäure |
| 0,1 bis | 2 Gew.-% | 2-Naphthylamin-4,6,8-trisulfonsäure |
| 0,1 bis | 2 Gew.-% | 2-Naphthylamin-3,6,8-trisulfonsäure |

Naphthylamin-trisulfonsäure-Isomerengemische können neben den Naphthylamintrisulfonsäuren noch weitere Produkte enthalten. Solche Produkte können insbesondere Nebenprodukte, Zersetzungsprodukte oder nicht umgesetzte Zwischenprodukte aus den Herstellungsstufen für Naphthylamin-trisulfonsäure sein, beispielsweise Naphthalin-di-, -tri- und -tetra-sulfonsäuren, Nitronaphthalin-mono-, -di und -trisulfonsäuren, Naphthylamin-mono- und -disulfonsäuren, z.B. 1-Naphthylamin-3,6- und -5,7-disulfonsäure, weiterhin Dinaphthylsulfon-sulfosäuren und deren Amino- und Nitroderivate, sowie Oxidationsprodukte des Naphthalins und/oder der Naphthalinsulfonsäuren, die bei der Sulfonierung und/oder der Nitrierung gebildet werden können.

Die 1-Naphthylamin-3,6,8-trisulfonsäure oder die Naphthylamintrisulfonsäure-Isomerengemische können in freier Form, in Form von neutralen Salzen oder in Form von sauren Salzen eingesetzt werden. Auch Gemische, die freie Säuren und Salze enthalten, können verwendet werden. Sofern die 1-Naphthylamin-3,6,8-trisulfonsäure oder die Naphthylamin-trisulfonsäure-Isomerengemische ganz oder teilweise als Salze vorliegen, sind die Alkali- und Erdalkalisalze, insbesondere die Natrium- und Kaliumsalze, bevorzugt.

Zum Einsatz in das erfindungsgemäße Verfahren geeignete 1-Naphthylamin-3,6,8-trisulfonsäure oder Salze dieser Säure können erhalten werden, indem man Naphthalin trisulfiert, das entstandene Gemisch nitriert, das dann vorliegende Nitro-Naphthalin-Trisulfonsäure-Gemisch reduziert, das saure Calcium-Natrium-Salz der T-Säure ausfällt, dieses Salz in Lösung mit Soda versetzt, die ausfallende Kreide abpreßt und die Salzlösung einengt. Diese Reaktionen können gemäß der eingangs beschriebenen Arbeitsweise nach FIAT Final Report oder auf beliebige andere Weise durchgeführt werden.

Ein zum Einsatz in das erfindungsgemäße Verfahren geeignetes Naphthylamin-trisulfonsäure-Isomerengemisch kann auf ähnliche Weise erhalten werden, wenn man die Reaktionsfolge nach beendeter Reduzierung des Nitro-naphthalintrisulfonsaure-Gemisches abbricht.

Die 1-Naphthylamin-3,6,8-trisulfonsäure und/oder deren Salze und/oder das Naphthylamin-trisulfonsäure-Isomerengemisch und/oder dessen Salze können beispielsweise in fester Form oder als wäßrige Lösung mit einem Gehalt von beispielsweise 20 bis 50 Gew.-%, vorzugsweise 30 bis 40 Gew.-%, gerechnet als freie Säure mit dem Molekulargewicht 383, eingesetzt werden.

Als Alkalihydroxidlösungen für das erfindungsgemäße Verfahren kommen insbesondere wäßrige Kali- oder Natronlauge in Frage. Der einsatz von Kalilauge führt im Vergleich zu Natronlauge zu besseren Ausbeuten, Natronlauge ist jedoch im allgemeinen kostengünstiger. Pro Mol diazotierbarer Substanz (gerechnet mit Molekulargewicht 383 = T-Säure) können beispielsweise 2,5 bis 12 Mol Alkalihydroxid eingesetzt werden. Besonders bevorzugt ist der Einsatz von 6 bis 9 Mol Alkalihydroxid pro Mol diazotierbarer Substanz. Die Konzentration von Alkalihydroxid im Reaktionsgemisch kann beispielsweise 10 bis 50 Gew.-% (bezogen auf die Summe Alkalihydroxid plus Wasser plus Alkohol) betragen. Bevorzugt ist diese Konzentration 25 bis 35%ig.

Ein wesentlicher Aspekt des erfindungsgemäßen Verfahrens ist, daß es in Gegenwart von Alkoholen und/oder Alkoholaten und in Gegenwart von Salpetersäure, Salpetriger Säure, Stickstoffoxiden, Nitraten, Nitriten, Nitroverbindungen oder Nitrosoverbindungen durchgeführt wird. Die zuzusetzenden Alkohole können dem Reaktionsgemisch beispielsweise in reiner Form, im Gemisch mit Wasser oder in Form von Alkoholaten, beispielsweise in Form von Alkalialkoholaten zugesetzt werden. Als Alkohole sind solche alkoholische Verbindungen geeignet, die unter Reaktionsbedingungen mit Waser mischbar sind und mit starkem Alkali keine oder nur in geringem Umfang unerwünschte Nebenreaktionen eingehen. Die Alkoholatbildung ist in diesem Falle keine unerwünschte Nebenreaktion. Bevorzugt kommen aliphatische Alkohole mit beispielsweise 1—6 Kohlenstoffatomen in Betracht. Es können beispielsweise primäre, sekundäre und tertiäre ein- und mehrwertige Alkohole verwendet werden, deren Hydroxygruppen auch ganz oder teilweise veräthert sein können. Als einwertige Alkohole kommen z.B in Frage: Methanol, Äthanol, n-Propanol, 2-Propanol, n-Butanol, Iso-butanol, tert.-Butanol. Als mehrwertige Alkohole kommen z.B. in Frage: Athylenglykol, Propandiole, Butandiole, Glycerin, Butantriole, Monoglyme, Diglyme, Selbstverständlich können auch Gemische von Alkoholen eingesetzt werden. Besonders bevorzugt kommt Methanol zum Einsatz.

Die Menge des einzusetzenden Alkohols bzw. Alkoholats kann beispielsweise so gewählt werden, daß 10 bis 80 Gew.-%, vorzugsweise 25 bis 60 Gew.-% Alkohol bzw. Alkoholat, bezogen auf die Summe Wasser plus Alkohol, vorliegen.

Als Stickstoffoxide kommen z.B. $N_2O_5$, $N_2O_4$, $N_2O_3$, $NO_2$ und $NO$, vorzugsweise $N_2O_5$ und $N_2O_4$ bzw. $NO_2$ in Frage. Als Nitrate kommen z.B. Alkalinitrate, Ammoniumnitrate, Erdalkalinitrate und sonstige Metallnitrate, auch Schwermetallnitrate, in Frage. Bevorzugt werden Alkalinitrate, insbesondere Natrium- oder Kaliumnitrat, eingesetzt. Als Nitrite kommen z.B. Alkalinitrite, insbesondere Natriumnitrit, in Frage. Als Nitroverbindungen kommen z.B. aromatische und aliphatische Nitroverbindungen in Frage, wie Nitrobenzole, Nitronaphthaline, Nitrobenzolsulfonsäure, Nitronaphthalin-mono-, -di- und -trisulfonsäuren, Nitromethan, Nitroäthan und Nitropropan. Als Nitrosoverbindungen kommen z.B. Nitrosobenzole in Frage.

Vorzugsweise werden solche Verbindungen eingesetzt, die selbst bzw. deren Folgeprodukte unter den Bedingungen für die Ausfällung des H-Säure-Monoalkalisalzes löslich sind, wie Stickstoff/Sauerstoff-Säuren, Stickstoffoxide, Nitrate, Nitrite und in saurem Medium lösliche Nitroverbindungen. So brauchen keine besonderen Vorkehrungen zur Abtrennung dieser Zusätze getroffen zu werden, da diese bzw. deren Folgeprodukte dann in dem Abwasser, nach der Abtrennung der H-Säure als Monoalkalisalz, enthalten sind.

Besonders bevorzugt wird das erfindungsgemäße Verfahren in Gegenwart von Natriumnitrat oder Kaliumnitrat durchgeführt.

Die vorstehend beschriebenen Zusätze können in reiner Form oder als Lösung, beispielsweise als Lösung in Wasser, wäßrigem Alkali oder Alkohol, in das erfindungsgemäße Verfahren eingebracht werden. Selbstverständlich können auch 2 oder mehr der genannten Zusätze eingesetzt werden.

Im allgemeinen ist es ausreichend, wenn die oben erwähnten N—O—enthaltenden Substanzen, in geringen Mengen im Reaktionsgemisch vorliegen. Man kann beispielsweise soviel dieser Substanzen in das Reaktionsgemisch einbringen, daß bezogen auf 1 Mol eingesetzte 1-Naphthylamin-3,6,8-trisulfonsäure bzw. 1 Mol Naphthylamin-Trisulfonsäure-Isomerengemisch bzw. 1 Mol Salze dieser Säuren 0,005 bis 1 Mol dieser Substanzen vorliegen. Vorzugsweise liegen diese Substanzen in einer Menge von 0,01 bis 0,5 Mol, insbesondere in einer Menge von 0,01 bis 0,1 Mol (Bezug wie oben) vor.

Das erfindungsgemäße Verfahren kann beispielsweise bei Temperaturen von 150 bis 250°C, vorzugsweise bei 180 bis 220°C, in einem geschlossenen Gefäß durchgeführt werden. Der sich dabei einstellende Druck ist im allgemeinen völlig ausreichend, um das erfindungsgemäße Verfahren in befriedigender Weise durchzuführen. Selbstverständlich kann man das erfindungsgemäße Verfahren auch bei anderen Drucken durchführen als denjenigen, die sich in geschlossenen Gefäßen von selbst einstellen. Beispielsweise sind für das erfindungsgemäße Verfahren Drucke im Bereich von 5 bis 100 bar möglich.

Die Reaktionszeit hängt im wesentlichen von der Reaktionstemperatur und der Alkalihydroxid-Konzentration ab. Sie ist bei relativ hohen Reaktionstemperaturen und bei relativ hohen Alkalihydroxid-Konzentrationen kürzer und bei relativ niedrigen Reaktionstemperaturen und relativ niedrigen Alkalihydroxid-Konzentrationen länger und beträgt im allgemeinen 10 Minuten bis 10 Stunden. Beispielsweise werden bei einer Reaktionstemperatur von ca. 200°C und einer Alkalihydroxid-Konzentration von 30 Gew.-% mit einer Reaktionszeit von 45—60 Minuten gute Ergebnisse erhalten.

Die in das erfindungsgemäße Verfahren einzusetzenden Stoffe werden am zweckmäßigsten mit einer solchen Temperatur in das Reaktionsgefäß eingebracht, daß man nach Freiwerden der Mischungs- und gegebenenfalls der Neutralisationswärme die gewünschte Reaktionstemperatur vorliegen hat. Man kann die einzugebenden Stoffe auch bei tieferen Temperaturen zusammenbringen und im Reaktionsgefäß auf die gewünschte Reaktionstemperatur erhitzen.

Nach Beendigung der Reaktion und vor Abscheiden der H-Säure als Monoalkalisalz ist es vorteilhaft, das Reaktionsgemisch zu kühlen und/oder mit Wasser zu verdünnen. Man kann beispielsweise auf Temperaturen im Bereich 20 bis 150°C, vorzugsweise auf Temperaturen im Bereich 80 bis 120°C abkühlen. Die gegebenenfalls zuzusetzende Wassermenge richtet sich nach den Reaktionsbedingungen, z.B. der Art des Alkalihydroxides, dessen Menge und Konzentration, sowie nach der gegebenenfalls noch vorliegenden Menge Alkohol. Es ist vorteilhaft, die Wassermenge so zu wählen, daß das bei der Reaktion gebildete Alkalisulfit gelöst wird bzw. gelöst bleibt.

Die Abscheidung der H-Säure als Monoalkalisalz kann durch Ansäuern der Reaktionsmischung mit Mineralsäuren erfolgen.

Vorzugsweise verwendet man hierzu Schwefelsäure. Man gibt soviel Mineralsäure zu, daß sich das schwerlösliche Monoalkalisalz der H-Säure bildet. Durch entsprechende Wahl der Konzentration der Mineralsäure und/oder durch Zugabe von Wasser vor und/oder während der Zugabe der Mineralsäure wird zweckmäßigerweise dafür gesorgt, daß das sich bildende anorganische Salz, z.B. Natriumsulfat oder Kaliumsulfat, nicht ausfällt. Man kann beispielsweise gute Ergebnisse erhalten, wenn man zur Abscheidung der H-Säure als Monoalkalisalz einen pH-Wert im Bereich 0 bis 4, vorzugsweise 0,5 bis 2,5, einstellt und durch Verdünnung mit Wasser und/oder durch entsprechende Wahl der Konzentration der Mineralsäure, bezogen auf das Gewicht des in der Druckhydrolyse vorliegenden Gemisches, die 0,1- bis 5-fache, vorzugsweise die 0,5- bis 2-fache Menge Wasser einbringt. Die Abtrennung des Monoalkalisalzes der H-Säure kann auf übliche Weise, beispielsweise durch Filtration, erfolgen. Es ist vorteilhaft, vor der Abtrennung des Monoalkalisalzes der H-Säure die Temperatur durch Kühlung, beispielsweise durch Verdampfungskühlung, auf weniger als 80°C einzustellen und die Abtrennung bei einer Temperatur von weniger als 80°C vorzunehmen. Bevorzugt wird die Abtrennung bei einer Temperatur im Bereich von 20 bis 60°C vorgenommen.

Zur vollständigen Entfernung von Schwefeldioxid ist es vorteilhaft, nach Einstellung der Fällungsbedingungen und vor der Abtrennung des Monoalkalisalzes der H-Säure das angesäuerte und verdünnte Gemisch für einige Zeit, beispielsweise 0,5 bis 2 Stunden, am Rückfluß zu kochen oder unter Vakuum zu halten oder das Schwefeldioxid mit einem inerten Gas, z.B. Stickstoff, auszublasen.

Das nach der Abtrennung vorliegende Monoalkalisalz der H-Säure wäscht man üblicherweise mit Wasser und trocknet es, beispielsweise im Vakuum.

Die Abtrennung des Alkohols kann nach der Umsetzung während der Aufarbeitung des Reaktionsgemisches an verschiedenen Stellen vorgenommen werden. Es ist möglich, den Alkohol aus alkalischer, neutraler oder saurer Lösung, vor oder nach der Abtrennung der H-Säure als Monoalkalisalz, abzutrennen. Bevorzugt erfolgt die Abtrennung des Alkohols aus alkalischer oder neutraler Lösung und durch Destillation. Besonders bevorzugt ist es, den Alkohol direkt aus dem Reaktionsgemisch, gegebenenfalls nach Abkühlung und/oder Verdünnung mit Wasser, über eine Kolonne abzudestillieren. Im Falle des Einsatzes niedrig siedender Alkohole, beispielsweise Methanol, kann es ausreichend sein, die Destillation ohne äußere Wärmezufuhr durch Druckentspannung in Gang zu bringen. Falls sich der verwendete Alkohol bei tieferen Temperaturen als der Reaktionstemperatur aus dem Reaktionsgemisch entmischt, ist es auch möglich den Alkohol durch eine Phasentrennung nach Abkühlung des Reaktionsgemisches abzutrennen.

Der abgetrennte Alkohol wird vorzugsweise im erfindungsgemäßen Verfahren wiederverwendet. Es ist dann nur nötig, gegebenenfalls während der alkalischen Druckhydrolyse und/oder während der

**0 000 495**

Aufarbeitung verlorengegangene Alkoholanteile zu ergänzen.

Das erfindungsgemäße Verfahren liefert gegenüber den bekannten Verfahren zur Herstellung von 1-Amino-8-naphthol-3,6-disulfonsäure (H-Säure) als Monoalkalisalz den Vorteil, daß höhere Ausbeuten erzielt werden können und die Bildung von Nebenprodukten, insbesondere die Bildung von 1-Amino-6-naphthol-3,8-disulfonsäure (W-Säure) und die Bildung von 1,8-Dihydroxynaphthalin-3,6-disulfonsäure (Chromotrop-Säure) erheblich vermindert wird. Der verminderte Gehalt der in saurer Lösung schwer löslichen W-Säure führt außerdem dazu, daß sich das Monoalkalisalz der H-Säure ohne eine intensive, mit Ausbeuteverlusten verbundene Wäsche, in besonders reiner Form isolieren läßt.

Beispiele

Beispiel 1

In einem 2,7 1 Nickel-Autoklav werden 580 g eines Naphthylamintrisulfonsäure-Gemisches in Form der Trinatriumsalze (Gehalt 11,9 g Gesamtnitrit/100 g, 52,8 Gew.-% T-Säure MG 383; insgesamt 69 g Nitrit, 0,80 Mol T-Säure) folgender Zusammensetzung:

| 1-Naphthylamin-3,6,8-trisulfonsäure | 80,0% |
| 1-Naphthylamin-3,5,7-trisulfonsäure | 8,5% |
| 1-Naphthylamin-4,6,8-trisulfonsäure | 4,0% |
| 1-Naphthylamin-2,5,7-trisulfonsäure | 3,0% |
| 2-Naphthylamin-3,5,7-trisulfonsäure | 1,2% |
| 2-Naphthylamin-4,6,8-trisulfonsäure | 0,7% |
| 2-Naphthylamin-3,6,8-trisulfonsäure | 0,5% |

(%-Gehalte jeweils bezogen auf diazotierbare Substanz) das zusätzlich
0,3 Gew.-% 1-Naphthylamin-3,6-disulfonsäure-dinatriumsalz,
1,3 Gew.-% Naphthalin-1,3,6-trisulfonsäure-trinatriumsalz,
0,6 Gew.-% 1-Nitronaphthalin-3,6,8-trisulfonsäure-trinatriumsalz,
4,6 Gew.-% Wasser und quantitativ nicht bestimmbare Mengen an Amino- und Nitroderivaten der Dinaphthylsulfosulfosäuren und an Oxidationsprodukten des Naphthalins und der Naphthalin-trisulfonsäuren enthält,

325 g Wasser, 310 g Methanol und
    a) 8,5 g (0,1 Mol) Natriumnitrat
    b) 4,2 g (0,05 Mol) Natriumnitrat
    c) 0,8 g (0,01 Mol) Natriumnitrat
    c) 6,9 g (0,1 Mol) Natriumnitrit

vorgelegt und auf 210°C aufgeheizt. In einem 1,3 l Stahl-Autoklav werden 474 g 70 gew.-%ige Natronlauge (8,3 Mol NaOH) auf 210°C aufgeheizt und mit Stickstoff quantitativ in den 2,7 l Autoklaven gedrückt, wodurch bezogen auf die Summe Wasser plus Methanol eine 30 gew.-%ige Natronlauge entsteht. Dabei stellt sich eine Temperatur von 220°C ein. Die Reaktionsmischung wird 20 Minuten bei 220°C gehalten, so schnell wie möglich abgekühlt, mit ca. 500 g Wasser verdünnt, das Methanol über eine Kolonne abdestilliert und das vom Methanol befreite Reaktionsgemisch durch Hochdruck-Flüssigkeits-Chromatographie quantitativ analysiert.

Die erhaltenen Versuchsergebnisse gehen aus Tabelle 1 hervor:

5

TABELLE 1

| Beispiel | Produktverteilung im vom Methanol befreiten Reaktionsgemisch in Mol—% bezogen auf eingesetzte T-Säure | |
| --- | --- | --- |
| | H-Säure MG 319 | 1-Naphthylamin-3,6-disulfonsäure MG 303 |
| 1 a | 82,4 | 1,8 |
| 1 b | 81,6 | 2,2 |
| 1 c | 79,0 | 4,7 |
| 1 d | 78,7 | 5,3 |

Die vom Methanol befreiten Reaktionsgemische werden pH-kontrolliert bei pH 1 bis 1,5, mit ca. 1000 g 50 gew.-%iger Schwefelsäure angesäuert, zur vollständigen Entfernung von Schwefeldioxid eine Stunde unter Rückfluß erhitzt, unter Verdampfungskühlung auf 40°C abgekühlt und zwei Stunden bei 40°C gehalten. Das Produkt wird bei 40°C filtriert, mit insgesamt 500 g Wasser gewaschen und bei 80°C im Vakuum getrocknet. Die Ausbeuten, sowie die H-Säure-Qualität, welche durch Hochdruck-Flüssigkeitschromatographie ermittelt wurde, sind in Tabelle 2 aufgeführt.

TABELLE 2

| Beispiel | Ausbeute bezogen auf eingesetzte T-Säure Mol—% | Qualität des isolierten Festprodukts [+)] | | | | | | |
|---|---|---|---|---|---|---|---|---|
| | | H-Säure MG 319 Gew.—% | 1-Naphthylamin 3,6-disulfonsäure MG 303 Gew.—% | W-Säure MG 319 Gew.—% | Chromotrop-Säure MG 320 Gew.—% | T-Säure MG 383 Gew.—% | Wasser Gew.—% | Natrium-sulfat Gew.—% |
| 1 a | 80 | 80,0 | 0,1 | — | 0,5 | — | 11,9 | 1,7 |
| 1 b | 79,5 | 81,0 | 0,2 | — | 0,6 | — | 11,8 | 0,9 |
| 1 c | 78 | 81,1 | 0,5 | — | 0,5 | — | 11,5 | 0,7 |
| 1 d | 77 | 81,3 | 0,6 | — | 0,6 | — | 11,9 | — |

[+)] Die Gehalte der angegebenen Säuren sind auf freie Säuren berechnet. Tatsächlich liegen sie als Mononatriumsalze (H-Säure, W-Säure, 1-Naphthylamin-3,6-disulfonsäure) bzw. Dinatriumsalze (Chromotropsäure, T-Säure) vor.

### Beispiele 2a—2h

Weitere wie in Beispiel 1, jedoch ohne Analyse des vom Methanol befreiten Reaktionsgemisches, durchgeführte Reaktionen bei 200°C unter Variation der Reaktionsparameter:

1. Molverhältnis NaOH zu T-Säure-Isomerengemisch-Trinatrium-Salz
2. NaOH-Konzentration
3. Gewichtsverhältnis Wasser zu Methanol
4. Menge des Natriumnitratzusatzes
5. Reaktionszeit

ergeben die in Tabelle 3 zusammengestellten Ergebnisse.

### Beispiel 3a—3e

In einem 2,7 l Nickel-Autoklaven werden 580 g des in Beispiel 1 angegebenen Naphthylamintrisulfonsäure-Gemisches, 275 g Wasser, 280 g Methanol und die in der nachstehenden Tabelle angegebene Menge an weiterem Zusatz vorgelegt und auf 190°C aufgeheizt. In einem 1,3 l Stahl-Autoklaven werden 430 g 70 gew.-%ige Natronlauge (7,5 Mol NaOH) auf 190°C aufgeheizt und mit Stickstoff quantitativ in den 2,7 l Autoklaven gedrückt, wodurch bezogen auf Wasser plus Methanol eine 30 gew.-%ige Natronlauge entsteht. Dabei stellt sich eine Temperatur von 200°C ein. Die Reaktionsmischung wird 50 Minuten bei 200°C gehalten, so schnell wie möglich abgekühlt, mit ca. 500 g Wasser verdünnt, das Methanol über eine Kolonne abdestilliert und das vom Methanol befreite Reaktionsgemisch durch Hochdruck-Flüssigkeits-Chromatographie quantitativ analysiert.

TABELLE 3

| Beispiel | Molverhältnis NaOH zu T-Säure-Isomerengemisch (MG 383) | NaOH-Konzentration bezogen auf Wasser plus Methanol Gew.-% | Gew.–Verhältnis Wasser zu Methanol | Natriumnitrat-zusatz bezogen auf eingesetztes Naphthylaminsulfonsäure-Gemisch Mol–% | Zeit Min. | bezogen auf eingesetzte T-Säure Mol–% | H-Säure MG 319 Gew.–% | 1-Naphthylamin-3,6-disulfonsäure MG 303 Gew.–% | Chromotropsäure MG 320 Gew.–% | T-Säure MG 383 Gew.–% | $H_2O$ Gew.–% | $Na_2SO_4$ Gew.–% |
|---|---|---|---|---|---|---|---|---|---|---|---|---|
| | | | | | | **Reaktionsbedingungen → Ausbeute** | | **Qualität des isolierten Festproduktes [+)]** | | | | |
| 2 a | 8,1:1 | 30 | 3:2 | 10,0 | 60 | 79,5 | 79,9 | 0,1 | 0,4 | — | 12,1 | 2,4 |
| 2 b | 7,5:1 | 30 | 3:2 | 10,0 | 51 | 79 | 81,1 | 0,05 | 0,3 | — | 12,0 | 0,7 |
| 2 c | 8,3:1 | 30 | 3:2 | 7,5 | 45 | 81 | 81,3 | 0,05 | 0,4 | 0,1 | 11,9 | 0,7 |
| 2 d | 7,5:1 | 30 | 3:2 | 7,5 | 50 | 80 | 81,3 | 0,1 | 0,3 | — | 12,1 | 0,6 |
| 2 e | 7,5:1 | 25 | 3:2 | 7,5 | 70 | 80 | 81,1 | — | — | — | 12,6 | 0,6 |
| 2 f | 7,3:1 | 25 | 2:1 | 7,5 | 85 | 77,5 | 80,6 | 0,05 | 0,5 | — | 12,9 | 0,4 |
| 2 g | 7,5:1 | 30 | 3:2 | 5,0 | 52 | 80 | 81,9 | 0,2 | 0,3 | — | 11,7 | 0,5 |
| 2 h | 7,1:1 | 30 | 3:2 | 5,0 | 55 | 79 | 79,8 | 0,15 | 0,6 | — | 12,0 | 1,9 |

[+)] W-Säure (MG 319) konnte in keinem der Produkte nachgewiesen werden. Die Gehalte der angegebenen Säuren sind auf freie Säuren berechnet. Tatsächlich liegen sie in Form der in Tabelle 2 angegebenen Salze vor.

# 0 000 495

Die erhaltenen Versuchsergebnisse gehen aus der Tabelle 4 hervor:

TABELLE 4

| Beispiel | Weiterer Zusatz in Mol–% bezogen auf eingesetztes Naphthylamintri-sulfonsäure-Gemisch | in Form von | Produktverteilung in vom Methanol befreiten Reaktionsgemisch in Mol–% bezogen auf eing. T-Säure | |
|---|---|---|---|---|
| | | | H-Säure MG 319 | 1-Naphthylamin-3,6-disulfon-säure MG 303 |
| 3 a | 5,0 | 2-Nitro-benzolsulfon-säure, Na-Salz | 78,2 | 6,3 |
| 3 b | 5,0 | Nitro-T-Säure, Trinatrium-Salz | 79,4 | 5,4 |
| 3 c | 5,0 | Nitrobenzol | 80,5 | 4,0 |
| 3 d | 5,0 | Kupfernitrat | 83,9 | 0,8 |
| 3 e | 7,5 | Natriumnitrat | 84,3 | 0,5 |

## Beispiel 4

In einem 2,7 l Nickel-Autoklav werden 450 g (1 Mol) T-Säure-Trinatriumsalz (Gehalt 15,3 g Nitrit/100 g, 85,1 Gew.-% T-Säure MG 383; insgesamt 69 g Nitrit), 325 g Wasser, 310 g Methanol und 8,5 g (0,1 Mol) Natriumnitrat vorgelegt und auf 200°C aufgeheizt. In einem 1,3 l Stahl-Autoklav werden 474 g 70 gew.-%ige Natronlauge (8,3 Mol NaOH) auf 215°C aufgeheizt und mit Stickstoff quantitativ in den 2,7 l Autoklaven gedrückt, wodurch bezogen auf die Summe Wasser plus Methanol eine 30 gew.-%ige Natronlauge entsteht. Dabei stellt sich eine Temperatur von 220°C ein. Die Reaktionsmischung wird 15 Minuten bei 220°C gehalten, so schnell wie möglich abgekühlt, mit ca. 500 g Wasser verdünnt und das Methanol über eine Kolonne abdestilliert. Die heiße Reaktionslösung wird in eine heiße Mischung aus 325 ml 96%iger Schwefelsäure und 1,5 l Wasser einlaufen lassen. Die resultierende H-Säure-Suspension wird zur Entfernung von Schwefeldioxid eine Stunde unter Rückfluß gekocht, auf 40 bis 45°C abgekühlt und eine Stunde bei 40 bis 45°C gehalten. Das Produkt wird bei 40°C filtriert, mit insgesamt 500 g Wasser gewaschen und bei 80°C im Vakuum getrocknet. Die Ausbeute beträgt 82% der Theorie.

Gehalt [Gew.-%]:

| | |
|---|---|
| H-Säure (MG 319) | 80,4 |
| 1-Naphthylamin-3,6-disulfonsäure (MG 303) | — |
| W-Säure (MG 319) | — |
| Chromotrop-Sare (MG 320) | 0,5 |
| T-Säure (MG 383) | — |
| $H_2O$ | 12,3 |
| $Na_2SO_4$ | 1,2 |

Die Gehalte der angegebenen Säuren sind auf freie Säuren berechnet. Tatsächlich liegen die in Beispiel 1 genannten Salze vor.

## Beispiel 5

5,9 kg eines Naphthylamin-trisulfonsäure-Gemisches in Form der Trinatriumsalze (Gehalt 11,7 g

10

Gesamtnitrit/100 g, 53,9 Gew.-% T-Säure vom Molekulargewicht 383, insgesamt 0,69 kg Nitrit, 8,3 Mol T-Säure) folgender Zusammensetzung:

1-Naphthylamin-3,6,8-trisulfonsäure 83,0%
1-Naphthylamin-3,5,7-trisulfonsäure 7,3%
1-Naphthylamin-4,6,8-trisulfonsäure 3,4%
1-Naphthylamin-2,5,7-trisulfonsäure 3,2%
2-Naphthylamin-3,5,7-trisulfonsäure 0,8%
2-Naphthylamin-4,6,8-trisulfonsäure 0,3%
2-Naphthylamin-3,6,8-trisulfonsäure 0,5%

(Prozentgehalte jeweils bezogen auf diazotierbare Substanz), das zusätzlich
0,2 Gew.-% 1-Naphthylamin-3,6-disulfonsäure-dinatriumsalz
1,1 Gew.-% Naphthylamin-1,3,6-trisulfonsäure-trinatriumsalz
5,4 Gew.-% Wasser
und quantitativ nicht bestimmbare Mengen an Amino- und Nitroderivaten der Dinaphthylsulfon-sulfosäure und an Oxidationsprodukten des Naphthalins und der Naphthalin-trisulfonsäuren enthält, sowie

2,8 kg Wasser, 2,8 kg Methanol und 64 g (0,75 Mol) Natriumnitrat werden in einem 20 l Nickel-Autoklaven auf 180°C aufgeheizt. Unter Stickstoff-überlagerung werden im Verlaufe von ca. 5 Minuten 4,3 kg 70 gew.-%ige Natronlauge 75 Mol NaOH) von 180°C eingepumpt, wobei sich eine Temperatur von 200°C einstellt. Die Reaktionsmischung wird 55 Minuten bei 200°C gehalten und in einem 25 l Edelstahl-Kessel entspannt, in dem 10 l kaltes Wasser vorgelegt sind. Das Methanol wird abdestilliert und die verdünnte H-Säure-Isomerengemisch-Lösung im Verlauf von 40 bis 60 Minuten mit Stickstoff in ein Fällungsgefäß aus Glas gedrückt, in dem ca. 4 kg Wasser (bzw. Waschwasser aus der Vorpartie) vorgelegt sind. Durch gleichzeitigen Zulauf von ca. 5,2 kg 100%iger Schwefelsäure wird der pH-Wert bei 1 bis 1,5 gehalten und die Reaktionsmischung zum Sieden gebracht. Die heiße, saure H-Säure-Suspension wird durch Anlegen von Vakuum vom restlichen Schwefeldioxid befreit, im Verlaufe von einer Stunde auf 40°C abgekühlt, eine Stunde bei 40°C gehalten und filtriert.

Das Produkt wird mit insgesamt 5,4 kg Wasser gewaschen und im Vakuum bei 80°C getrocknet. Die Ausbeute beträgt 82% bezogen auf T-Säure. Die durch Hochdruck-Flüssigkeits-Chromatographie ermittelten Qualitäten des Produktes sind in Tabelle 5 aufgeführt.

Beispiel 6

Eine wie in Beispiel 5 durchgeführte Reaktion, bei der jedoch die Reaktionstemperatur 190°C und die Reaktionszeit 100 Minuten beträgt, ergibt eine Ausbeute von 83%, bezogen auf T-Säure. Die durch Hochdruck-Flüssigkeits-Chromatographie ermittelte Qualität des Produktes ist in Tabelle 5 aufgeführt.

TABELLE 5

| | Qualität des isolierten Festprodukts [+] | | | | |
|---|---|---|---|---|---|
| Beispiel | H-Säure MG 319 Gew.-% | 1-Naphthylamin-3,6-disulfonsäure MG 303 Gew.-% | W-Säure MG 319 Gew.-% | Chromotrop-Säure MG 320 Gew.-% | T-Säure MG 383 Gew.-% |
| 5 | 81,3 | 0,1 | — | 0,7 | 0,3 |
| 6 | 80,8 | 0,1 | — | 1,0 | 0,2 |

[+] Ergänzend zu 100 % liegt praktisch nur noch Natriumsulfat und Wasser vor. Die angegebenen Gehalte der Säuren sind auf freie Säuren berechnet. Tatsächlich liegen diese in Form der in Beispiel 2 angegebenen Salze vor.

Beispiel 7 (Vergleichsbeispiel)

In einem 2,7 Liter Nickel-Autoklav werden 580 g eines Naphthylamintrisulfonsäure-Gemisches in Form der Trinatriumsalze (Gehalt 11,9 Gesamtnitrit/100 g, 52,8 Gew.-% T-Säure MG 383; insgesamt 69 g Nitrit, 0,80 Mol T-Säure) folgender Zusammensetzung:

1-Naphthylamin-3,6,8-trisulfonsäure 80,0%
1-Naphthylamin-3,5,7-trisulfonsäure 8.5%
1-Naphthylamin-4,6,8-trisulfonsäure 4,0%
1-Naphthylamin-2,5,7-trisulfonsäure 3,0%
2-Naphthylamin-3,5,7-trisulfonsäure 1,2%
2-Naphthylamin-4,6,8-trisulfonsäure 0,7%
2-Naphthylamin-3,6,8-trisulfonsäure 0,5%

(%-Gehalte jeweils bezogen auf diazotierbare Substanz) das zusätzlich
0,3 Gew.-% 1-Naphthylamin-3,6-disulfonsäure-dinatriumsalz,
1,3 Gew.-% Naphthalin-1,3,6-trisulfonsäure-trinatriumsalz,
0,6 Gew.-% 1-Nitronaphthalin-3,6,8-trisulfonsäure-trinatriumsalz,
4,6 Gew.-% Wasser und quantitativ nicht bestimmbare Mengen an Amino- und Nitroderivaten der Dinaphthylsulfon-sulfosäure und an Oxidationsprodukten des Naphthalins und der Naphthalin-tri-sulfosäuren enthält und 400 g Wasser vorgelegt und auf 180°C aufgeheizt. In einem 1,3 Liter Stahl-Autoklav werden 600 g 50 gew.-%ige Natronlauge (7,5 Mol NaOH) auf 185°C aufgeheizt und mit Stickstoff in den 2,7 Liter Autoklaven gedrückt, wodurch bezogen auf das gesamte Wasser eine 30 gew.-%ige Natronlauge entsteht. Dabei stellt sich eine Temperatur von 200°C ein. Die Reaktionsmischung wird 45 Minuten bei 200°C gehalten, so schnell wie möglich auf 100°C abgekühlt und mit 500 g Wasser verdünnt. Die heiße Reaktionslösung wird pH-kontrolliert (pH 1 bis 1,5) mit ca. 1000 g 50 gew.-%iger Schwefelsäure angesäuert, zur vollständigen Entfernung von Schwefeldioxid eine Stunde unter Rückfluß erhitzt, unter Verdampfungskühlung auf 40°C abgekühlt und 2 Stunden bei 40°C gehalten. Das Produkt wird bei 40°C filtriert, mit insgesamt 500 g Wasser gewaschen und bei 80°C im Vakuum getrocknet.

Die Ausbeute beträgt 58%, bezogen auf T-Säure-Isomerengemisch bzw. 72% bezogen auf T-Säure. Die H-Säure-Qualität wurde durch Hochdruck-Flüssigkeits-Chromatographie wie folgt ermittelt.

H-Säure-Mononatriumsalz 88,2%
1-Naphthylamin-3,6-disulfonsäure-Mononatriumsalz 0,1—0,2%
W-Säure-Mononatriumsalz 0,1—0,2%
Chromotropsäure-Dinatriumsalz 1,1—1,2%
T-Säure-Dinatriumsalz 0,1—0,2%
Wasser 9,0%
Natriumsulfat 0,5%

Reaktionsprodukte aus den isomeren Naphthylamintrisulfonsäuren sind in dem isolierten Produkt nicht enthalten.

Beispiel 8 (Vergleichsbeispiel)

Eine wie in Beispiel 7 durchgeführt Reaktion, jedoch unter Einsatz von reinem T-Saure-Trinatriumsalz ergibt eine Ausbeute von 73%, jedoch einen erhöhten Gehalt von W- und T-Säure.

Gehalt:

H-Säure-Mononatriumsalz 88,6%
1-Naphthylamin-3,6-disulfonsäure-Mononatriumsalz 0,2%
W-Säure-Mononatriumsalz 2,0%
Chromotropsäure-Dinatriumsalz 1,4%
T-Säure-Dinatriumsalz 0,6%
Wasser 6,8%
Natriumsulfat 0,6%

**Patentansprüche**

1. Verfahren zur Herstellung von 1-Amino-8-naphthol-3,6-disulfonsäure-Monoalkalisalzen durch Umsetzung von 1-Naphthylamin-3,6,8-trisulfonsäure und/oder deren Salzen und/oder Naphthylamin-trisulfonsäure-Isomerengemischen und/oder deren Salzen mit Alkalihydroxidlösung bei erhöhtem Druck und erhöhter Temperatur und Abscheidung von 1-Amino-8-naphthol-3,6-disulfonsäure-Monoalkali-salzen durch Ansäuern, dadurch gekennzeichnet, daß man die Umsetzung in Gegenwart von Alkoholen und/oder Alkoholaten und in Gegenwart von Salpetersäure, salpetriger Säure, Stickstoffoxiden, Nitraten, Nitriten, Nitroverbindungen oder Nitrosoverbindungen durchführt.

2. Verfahren nach Anspruch 1, dadurch gekennzeichnet, daß man die Umsetzung in Gegenwart aliphatischer Alkohole mit 1 bis 6 Kohlenstoffatomen durchführt.

3. Verfahren nach Anspruch 1 und 2, dadurch gekennzeichnet, daß man die Umsetzung in Gegenwart von Methanol durchführt.

4. Verfahren nach Anspruch 1 bis 3, dadurch gekennzeichnet, daß während der Umsetzung 10 bis

**0 000 495**

80 Gew.-% Alkohol und/oder Alkoholate, bezogen auf die Summe Wasser plus Alkohol, vorliegen.

5. Verfahren nach Anspruch 1 bis 4, dadurch gekennzeichnet, daß man das Verfahren in Gegenwart von Natriumnitrat oder Kaliumnitrat durchführt.

6. Verfahren nach Anspruch 1 bis 5, dadurch gekennzeichnet, daß man 0,005 bis 1 Mol Salpetersäure, salpetrige Säure, Stickstoffoxide, Nitrate, Nitrite, Nitroverbindungen oder Nitrosoverbindungen je Mol eingesetzte 1-Naphthylamin-3,6,8-trisulfonsäure bzw. pro Mol Naphthylamin-trisulfonsäure-Isomerengemisch bzw. pro Mol Salze dieser Säuren in das Reaktionsgemisch einbringt.

7. Verfahren nach Anspruch 1 bis 6, dadurch gekennzeichnet, daß man die Umsetzung bei Temperaturen von 150 bis 250°C durchführt.

8. Verfahren nach Anspruch 1 bis 7, dadurch gekennzeichnet, daß man die Umsetzung in einem geschlossenen Gefäß unter dem sich einstellenden Druck durchführt.

9. Verfahren nach Anspruch 1 bis 7, dadurch gekennzeichnet, daß man die Umsetzung bei einem Druck von 5 bis 100 bar durchführt.

10. Verfahren nach Anspruch 1 bis 9, dadurch gekennzeichnet, daß man nach der Umsetzung den Alkohol aus alkalischer oder neutraler Lösung durch Destillation abtrennt.

11. Verfahren nach Anspruch 10, dadurch gekennzeichnet, daß man den abgetrennten Alkohol wieder verwendet.

**Revendications**

1. Procédé de production de sels mono-alcalins d'acide 1-amino-8-naphtol-3,6-disulfonique par réaction d'acide 1-naphtylamine-3,6,8-trisulfonique et/ou de ses sels et/ou de mélanges d'isomères d'acide naphtylamine-trisulfonique et/ou de leurs sels avec une solution d'hydroxide alcalin sous pression élevée et à température élevée et précipitation des sels mono-alcalins d'acide 1-amino-8-naphtol-3,6-disulfonique par acidification, caractérisé en ce qu'on conduit la réaction en présence d'alcools et/ou d'alcoolates et en présence d'acide nitrique, d'acide nitreux, d'oxydes d'azote, de nitrates, de nitrites, de composés nitrés ou de composés nitroso.

2. Procédé suivant la revendication 1, caractérisé en ce qu'on conduit la réaction en présence d'alcools aliphatiques ayant 1 à 6 atomes de carbone.

3. Procédé suivant les revendications 1 et 2, caractérisé en ce qu'on conduit la réaction en présence de méthanol.

4. Procédé suivant les revendications 1 à 3, caractérisé en ce que 10 à 80% en poids d'alcool et/ou d'alcoolate par rapport à la somme eau plus alcool sont présents pendant la réaction.

5. Procédé suivant les revendications 1 à 4, caractérisé en ce qu'il est mis en oeuvre en présence de nitrate de sodium ou de nitrate de potassium.

6. Procédé suivant les revendications 1 à 5, caractérisé en ce qu'on introduit dans le mélange réactionnel 0,005 à 1 mole d'acide nitrique, d'acide nitreux, d'oxydes d'azote, de nitrates, de nitrites, de composés nitrés ou de composés nitroso par mole d'acide 1-naphtylamine-3,6,8-trisulfonique utilisé ou par mole de mélange d'isomères d'acide naphtylamine-trisulfonique utilisé ou par mole de sels de ces acides.

7. Procédé suivant les revendications 1 à 6, caractérisé en ce qu'on conduit la réaction à des températures de 150 à 250°C.

8. Procédé suivant les revendications 1 à 7, caractérisé en ce qu'on conduit la réaction sous pression autogène dans un récipient clos.

9. Procédé suivant les revendications 1 à 7, caractérisé en ce qu'on conduit la réaction à une pression de 5 à 100 bars.

10. Procédé suivant les revendications 1 à 9, caractérisé en ce qu'on sépare l'alcool de la solution alcaline ou neutre par distillation après la réaction.

11. Procédé suivant la revendication 10, caractérisé en ce qu'on réutilise l'alcool séparé.

**Claims**

1. A process for the preparation of mono-alkali metal salts of 1-amino-8-naphthol-3,6-disulphonic acid by reacting 1-naphthylamine-3,6,8-trisulphonic acid and/or the salts thereof and/or naphthylamine-trisulphonic acid isomer mixture and/or the salts thereof with an alkali metal hydroxide solution at elevated pressure and elevated temperature, and separating out the mono-alkali metal salts of 1-amino-8-naphthol-3,6-disulphonic acid by acidification, characterised in that the reaction is carried out in the presence of alcohols and/or alcoholates and in the presence of nitric acid, nitrous acid, nitrogen oxides, nitrates, nitrites, nitro compounds or nitroso compounds.

2. A process acccording to claim 1, characterised in that the reaction is carried out in the presence of aliphatic alcohols having 1 to 6 carbon atoms.

3. A process according to claim 1 and 2, characterised in that the reaction is carried out in the presence of methanol.

4. A process according to claim 1 to 3, characterised in that 10 to 80% by weight of alcohol and/or alcoholate, relative to the sum of water plus alcohol, is present during the reaction.

0 000 495

5. A process according to claim 1 to 4, characterised in that the process is carried out in the presence of sodium nitrate or potassium nitrate.

6. A process according to claim 1 to 5, characterised in that from 0.005 to 1 mol of nitric acid, nitrous acid, nitrogen oxides, nitrates, nitrites, nitro compounds or nitroso compounds is introduced into the reaction mixture per mol of 1-naphthylamine-3,6,8-trisulphonic acid used or per mol of naphthylamine-trisulphonic acid isomer mixture, or per mol of the salts of these acids.

7. A process according to claim 1 to 6, characterised in that the reaction is carried out at temperatures from 150 to 250°C.

8. A process according to claim 1 to 7, characterised in that the reaction is carried out in a closed vessel under the pressure which automatically forms.

9. A process according to claim 1 to 7, characterised in that the reaction is carried out under a pressure of from 5 to 100 bars.

10. A process according to claim 1 to 9, characterised in that after the reaction the alcohol is separated off from the alkaline or neutral solution by distillation.

11. A process according to claim 10, characterised in that the alcohol which is separated off is reused.

14